Europäisches Patentamt

European Patent Office

Office européen des brevets

(19)

(11) Veröffentlichungsnummer: **0 278 487**

**A2**

(12) **EUROPÄISCHE PATENTANMELDUNG**

(21) Anmeldenummer: 88101926.9

(51) Int. Cl.⁴: **C12N 7/06**

(22) Anmeldetag: 10.02.88

(30) Priorität: 13.02.87 DE 3704550

(43) Veröffentlichungstag der Anmeldung:
17.08.88 Patentblatt 88/33

(84) Benannte Vertragsstaaten:
AT BE CH DE ES FR GB GR IT LI LU NL SE

(71) Anmelder: **BEHRINGWERKE AKTIENGESELLSCHAFT**
**Postfach 1140**
**D-3550 Marburg/Lahn(DE)**

(72) Erfinder: **Bosslet, Klaus, Dr.**
**Am Schlag 5**
**D-3550 Marburg(DE)**
Erfinder: **Hilfenhaus, Joachim, Dr.**
**Auf'm Gebrande 24**
**D-3550 Marburg(DE)**

(74) Vertreter: **Meyer-Dulheuer, Karl-Hermann, Dr.**
**et al**
**HOECHST Aktiengesellschaft Zentrale**
**Patentabteilung Postfach 80 03 20**
**D-6230 Frankfurt/Main 80(DE)**

(54) Verfahren zur Inaktivierung hüllhaltiger Viren in mittels einer Zelle in vitro hergestellten Protein-Präparationen.

(57) Es wird ein Verfahren zur Inaktivierung hüllhaltiger Viren in Proteinpräparationen, die in vitro mittels einer Zelle und besonders hybridom-oder gentechnisch hergestellt wurden, durch Behandeln mit einem nicht-ionischen Detergenz, worunter auch ein zwitter-ionisches verstanden wird, beschrieben.

EP 0 278 487 A2

# 0 278 487

## Verfahren zur Inaktivierung hüllhaltiger Viren in mittels einer Zelle in vitro hergestellten Protein-Präparationen

Die Erfindung betrifft ein Verfahren zur Inaktivierung von hüllhaltigen Viren in Präparationen von Proteinen, die mittels einer Zelle in vitro und besonders mit der Hybridom-oder Gentechnik hergestellt wurden, durch Behandlung mit nicht-ionischen Detergenzien, wobei die biologische Aktivität der Proteine erhalten bleibt.

Solche Proteinpräparationen sind beispielsweise solche von monoklonalen Antikörpern. Monoklonale Antikörper produzierende Zellinien werden durch die Fusionierung von primären B Lymphozyten mit geeigneten, permanent vermehrbaren Zellinien zu sogenannten Hybridomas oder zur Gewinnung menschlicher monoklonaler Antikörper durch Immortalisierung von primären menschlichen B-Lymphozyten durch Epstein-Barr-Virus gewonnen. Zur Fusionierung geeignete Zellinien von der Maus enthalten häufig murine Retro-oder andere hüllhaltige Viren, die in der Regel auch in dem resultierenden Hybridom nachgewiesen werden können. In humanen monoklonale Antikörper produzierenden Zellinien, die durch Epstein-Barr-Virusinfektion gewonnen wurden, können Epstein-Barr-Viruspartikel auftreten.

Für die Zulassung von monoklonalen Antikörpern als Therapeutika wird gefordert, daß Antikörperpräparationen, die aus virus-kontaminierten Zellinien stammen, einem Ver fahren zur Virusinaktivierung oder -eliminierung unterworfen werden.

In EP-A-0 099 445 ist beschrieben, daß Hepatitis-Viren in Plasma oder -derivaten durch eine Behandlung mit Alkoholen, Ethern oder nicht-ionischen Detergenzien inaktiviert werden können.

Überraschenderweise wurde gefunden, daß die nichtionischen Detergenzien [R]Triton X 100 und [R]Nonidet P-40 bei niedriger Temperatur beispielsweise 4°C, und in einer Konzentration von beispielsweise gleich oder kleiner 1 ml/100 ml hüllhaltige Viren in einer Antikörperlösung inaktivierten, ohne die Immunreaktivität der Antikörper zu beeinträchtigen.

Gegenstand der Erfindung ist deshalb ein Verfahren zur Inaktivierung hüllhaltiger Viren in einer Lösung eines Proteins, das in vitro mittels einer Zelle hergestellt wurde, dadurch gekennzeichnet, daß dieser Lösung ein nicht-ionisches Detergenz zugesetzt und die Mischung stehen gelassen wird.

Verfahren zur Herstellung von Proteinen mittels Zellen in vitro sind beispielsweise hybridom-oder gentechnische Verfahren.

Hüllhaltige Viren im Sinne der Erfindung sind vor allem Retroviren und Epstein-Barr-Viren.

Ein solches nicht-ionisches Detergenz ist vorzugsweise eines auf der Basis von Polyoxyethylen, beispielsweise Ether und Ester von einem solchen, und besonders [R]Triton X-100 oder [R]Nonidet P-40.

Ein solches Detergenz ist aber auch ein zwitter-ionisches in einem pH-Bereich, in welchem es keine äußere Ladung aufweist.

Es wird höchstens in einer Konzentration verwendet, daß die biologische Aktivität nicht wesentlich beeinträchtigt wird, das heißt bis 2 g/100 ml und vorzugsweise 0,4 bis 0,6 ml/100 ml Antikörperlösung.

Es wird bei einer Temperatur über dem Gefrierpunkt bis Raumtemperatur, vorzugsweise bei 2 bis 10°C gearbeitet.

Das Detergenz wird so lange einwirken gelassen, bis kein infektiöses Virus nachweisbar ist. Die Behandlungszeit beträgt mindestens eine Minute. Aber auch längere Inkubationszeiten von beispielsweise 24 Stunden sind möglich, wenn dadurch die Immunreaktivität der Antikörper nicht wesentlich beeinträchtigt wird. Herpes simplex-oder Rous-Sarkom-Viren sind bereits nach einstündiger Inkubation vollständig inaktiviert. Dieses bedeutet, daß zwar eine einstündige Inkubation bei 4°C in Gegenwart eines nicht-ionischen Detergenzes ausreicht hüllhaltige Viren abzutöten, daß aber, sofern eine erhöhte Sicherheit gefordert wird, längere Inkubationszeiten möglich sind.

Dieses Verfahren ist geeignet, empfindliche Proteine frei von infektiösen, hüllhaltigen Viren herzustellen, wie hybridom-oder gentechnisch in Zellkulturen gewonnene Proteine.

Beispiele

1. Inaktivierung von Herpes simplex Virus in einer Antikörperpräparation

Da Epstein-Barr-Virus schwer züchtbar und nur in geringen Konzentrationen gewinnbar ist, wurde ein anderes Herpesvirus verwendet, das in hohen Konzentrationen gewinnbar ist und mit dem folglich die hohe Inaktivierungsrate des beanspruchten Verfahrens gezeigt wird.

47,5 ml einer monoklonalen Antikörperpräparation (MAK) wurden mit 2,5 ml einer 100 ml/l wässrigen

2

RTriton X 100-Lösung bei 4°C gemischt. 45 ml dieser Mischung wurden mit 5 ml einer Herpes simplex Viruspräparation gemischt (2 in Tabelle 1). Dann wurde bis zu maximal 8 Stunden bei 4°C inkubiert. Nach 1, 4 und 8 Stunden wurden Proben zur Virustitration entnommen, sofort 1:10 mit eiskaltem Zellkulturmedium verdünnt und bis zur Testung bei -80°C eingefroren. Parallel zur detergenzhaltigen Mischung wurde eine Virusprobe 1:10 mit der monoklonalen Antikörperpräparation verdünnt (1) und diese Mischung ebenfalls 8 Stunden bei 4°C inkubiert. Diese diente als Kontrolle.

Die Ergebnisse sind in Tabelle 1 zusammengefaßt.

## Tabelle 1:

Inaktivierung von Herpes simplex Virus in einer monoklonalen Antikörperpräparation (Inkubation mit RTriton X 100 bei 4°C)

Virustiter ($\log_{10} ID_{50}$/ml)

|  | 0 Std | 1 Std | 4 Std | 8 Std |
|---|---|---|---|---|
| Virus + MAK (1) | 5,44 | n.d.[*] | n.d. | 5,40 |
| Virus + MAK + 0,5% Triton X 100 (2) | 5,44[**] | neg.[***] | neg. | n.d. |

[*] n.d. = nicht durchgeführt

[**] Titer vor Triton X 100-Zugabe auf Grund des Titers der Viruspräparation berechnet

[***] $2 \times 1$ ml der Probe in 100 ml Medium verdünnt war in suszeptiblen Zellkulturen frei von infektiösem Virus.

## 2. Inaktivierung von Rous-Sarkom-Virus

Da die bisher in Hybridomzellen nachgewiesenen Retroviren in vitro nicht ausreichend vermehrbar sind, wurde für diese Untersuchungen ein aviäres Retrovirus, das Rous-Sarkom-Virus, eingesetzt, das in geeignetem Zellsystem zu hochtitrigen Präparationen vermehrt und als infektiöses Virus in geeigneten Testsystemen quantitativ nachweisbar ist.

Die Testbedingungen entsprachen denen für Herpes-simplex-Virus (Beipiel 1). Die Verdünnung war jedoch 1:5. Die Ergebnisse sind in der nachfolgenden Tabelle 2 zusammengefaßt.

## Tabelle 2.:

Inaktivierung von Rous-Sarkom-Virus in einer monoklonalen Antikörperpräparation

Virustiter ($\log_{10} ID_{50}$/ml)

|  | 0 Std | 4 Std | 8 Std |
|---|---|---|---|
| Virus | 4,9 | n.d.[*] | n.d. |
| Virus + MAK + Triton X 100 | 4,9[**] | neg.[***] | neg. |

[*] n d = nicht durchgeführt
[**] Titer vor [R]Triton X-100-Zugabe berechnet auf Grund des Titers der Viruspräparation
[***] 2 × 1 ml der Probe in 100 ml Medium verdünnt war in suszeptiblen Zellkulturen frei von infektiösem Virus

3. Immunreaktivität mit Detergenz behandelter monoklonaler Antikörper

Präparation der monoklonalen Antikörper BW 494/32 und 495/36 (EP 86 111 708.3) sowie 431/26, 431/31, 250/183 und 374/14 (EP-A-0 160 897) wurden wie folgt behandelt:

Jeweils 4,75 ml Hybridomüberstand (MAk Konzentration = 20 μg/ml) wurden mit je 250 μl einer 100 ml/l wässrigen [R]Triton X-100 Lösung gemischt und 18 Stunden bei 4°C inkubiert. Nach 1, 4, 8 und 18 Stunden wurden Proben entnommen und die Fähigkeit jedes MAk, mit seinem spezifischen Antigen zu reagieren, quantitativ im Bio-Dot Assay bestimmt (Int. J. Cancer 36, (1985) 75-84). Hierzu wurden je 200 μl Aliquots der mit [R]Triton X-100 inkubierten MAk-Lösungen in einer Verdünnungsreihe als Erstantikörper eingesetzt und ihre spezifische Immunreaktivität bestimmt.

Es zeigte sich, daß die Bindung aller geprüften MAk (außer MAk 494/32) in geringer Verdünnung an das entsprechende spezifische Antigen im Beisein von Triton X-100 gehemmt wurde, daß aber bei weiterem Verdünnen die Immunreaktivität wieder voll erschien (vergleichbar mit Kontrolle ohne Tritonzugabe). Die Inkubation mit Detergenz bewirkt also nur eine reversible Blockierung der Immunreaktivität.

Die spezifische Immunaktivität der zur Inaktivierung von Viren mit Detergenz behandelten monoklonalen Antikörper ist nicht beeinträchtigt.

## Ansprüche

1. Verfahren zur Inaktivierung hüllhaltiger Viren in einer Lösung eines Proteins, das in vitro mittels einer Zelle hergestellt wurde, dadurch gekennzeichnet, daß dieser Lösung ein nicht-ionisches Detergenz zugesetzt und die Mischung stehen gelassen wird.

2. Verfahren nach Anspruch 1, dadurch gekennzeichnet, daß als nicht-ionisches Detergenz ein zwitterionisches verwendet wird.

3. Verfahren nach Anspruch 1, dadurch gekennzeichnet, daß das Protein hybridom-oder gentechnisch hergestellt wurde.

4. Verfahren nach Anspruch 1, dadurch gekennzeichnet, daß das Detergenz ein gegebenenfalls verethertes oder verestertes Polyoxyethylen ist.

5. Verfahren nach Anspruch 1, dadurch gekennzeichnet, daß das Detergenz [R]Triton X-100 oder [R]Nonidet P-40 ist.

6. Verfahren nach Anspruch 1, dadurch gekennzeichnet, daß das Detergenz in einer Konzentration von bis zu 2 g/100 ml, vorzugsweise 0,4 bis 0,6 ml/100 ml Antikörperlösung verwendet wird.

7. Verfahren nach Anspruch 1, dadurch gekennzeichnet, daß die das Detergenz enthaltende Lösung bei einer Temperatur über dem Gefrierpunkt bis Raumtemperatur, vorzugsweise 2 bis 10°C stehen gelassen wird.

8. Verfahren nach Anspruch 1, dadurch gekennzeichnet, daß die das Detergenz enthaltende Lösung eine Minute bis 48 Stunden stehen gelassen wird.